**Europäisches Patentamt**

**European Patent Office**

**Office européen des brevets**

(19)

(11) Numéro de publication: **0 049 666**
**B1**

(12)     **FASCICULE DE BREVET EUROPEEN**

(45) Date de publication du fascicule du brevet:
01.02.84

(51) Int. Cl.³: **A 61 K 37/18** // A61K37/16

(21) Numéro de dépôt: 81401537.6

(22) Date de dépôt: 02.10.81

(54) **Nouvelles substances biologiquement actives, leur obtention à partir de caséine humaine et compositions les contenant.**

(30) Priorité: 03.10.80 FR 8021292

(43) Date de publication de la demande:
14.04.82 Bulletin 82/15

(45) Mention de la délivrance du brevet:
01.02.84 Bulletin 84/5

(84) Etats contractants désignés:
AT BE CH DE FR GB IT LI LU NL SE

(56) Documents cités:
FR - A - 1 411 478
FR - A - 1 461 423
FR - A - 1 466 141
US - A - 3 558 770

(73) Titulaire: **INSTITUT NATIONAL DE LA SANTE ET DE LA RECHERCHE MEDICALE (INSERM), 101, rue de Tolbiac, F-75654 Paris Cedex 13 (FR)**

(72) Inventeur: **Jolles, Pierre, 2, rue Jean-François Gerbillon, F-75270 Paris Cedex 06 (FR)**
Inventeur: **Migliore-Samour, Danièle, 64, avenue Charles Gide, F-94270 Le Kremlin Bicetre (FR)**

(74) Mandataire: **Phélip, Bruno et al, c/o Cabinet Harlé & Phélip 21, rue de la Rochefoucauld, F-75009 Paris (FR)**

Nouvelles substances biologiquement actives, leur obtention à partir de caséine
humaine et compositions les contenant

La présente invention concerne des substances biologiquement actives obtenues par fractionnement d'hydrolysats enzymatiques de la caséine humaine et les compositions qui les contiennent.

On a déjà proposé l'utilisation de dérivés de caséine dans des compositions biologiques. A cet effet on peut citer les brevets ci-après:

— le brevet FR 1 461 423 qui concerne une composition biologique destinée aux soins de la peau humaine; cette composition comporte, soit sous forme acide, soit sous forme de sels, la combinaison chimique obtenue par association du groupe carbonyle issu du groupe carboxyle de l'acide palmitique, aux fonctions aminées, imidazoliques, phénoliques et hydroxylées des fractions des caséines hydrolysées par voie chimique ou enzymatique.

— le brevet FR 1 466 141 qui concerne des substances biologiques destinées à la préparation de compositions détergentes à usage corporal. Ces compositions détergentes sont caractérisées par la présence soit, d'aminoacides acylés, soit de peptides acylés, soit du mélange des deux. Parmi les peptides acylés appropriés sont cités notamment les cides caséiniques substitués par un résidu d'acide gras.

— le brevet US 3 558 770 relatif à des compositions pour le traitement des blessures, qui contiennent une caséine modifiée par l'action d'une enzyme.

— le brevet FR 1 411 478 qui concerne une composition destinée à la protection de la peau contre l'agressivité des solvants et substances hydrophobes variées. Cette composition comporte une substance gélifiante d'origine animale (par exemple de la caséine), ou végétale ou purement chimique, un hydrolysat partiel ou total de protéines animales ou végétales, une substance à caractère tensio-actif et une faible quantité d'un détergent chimique.

On peut également citer à titre de documents illustrant l'état de la technique les brevets FR 1 518 665 et 2 422 400.

On notera que toutes les compositions décrites dans les brevets ci-dessus sont des compositions essentiellement à usage topique, notamment à des fins cosmétiques ou d'hygiène de la peau, dans lesquelles sont présents des dérivés de caséine, obtenus par exemple par hydrolyse enzymatique, lesdits dérivés étant couplés à des fractions lipidiques par voie de synthèse chimique. Ce couplage lipidique assure aux dérivés de caséine l'activité topique désirée. Rien n'indique que ces dérivés proviennent de caséine humaine et que le fractionnement de ces dérivés, indépendamment de tout couplage avec une fraction lipidique, conduirait à des substances à activité immunologique, c'est-à-dire à des substances dont le domaine d'application et le mode d'administration sont totalement différents de ceux décrits dans l'art antérieur ci-dessus.

L'invention présentement revendiquée permet d'obtenir, par fractionnement de caséine humaine ayant subi l'action d'une enzyme protéolytique, des substances biologiquement actives, qui sont des agents immunologiques favorisant en particulier la production d'anticorps.

La caséine humaine, qui est un complexe de protéines ($\alpha_5$, $\beta$, K, ...) est présente essentiellement dans le lait maternel. Sous l'action d'une enzyme protéolytique la caséine peut donner naissance, après fractionnement, à des substances biologiquement actives.

Selon la présente invention, les nouvelles substances sont obtenues par traitement, à l'aide d'au moins une enzyme protéolytique, de la caséine humaine délipidée puis solubilisée, suivi du fractionnement des produits hydrosolubles, en fonction de leur poids moléculaire moyen, par filtration sur un support approprié.

Les nouvelles substances selon l'invention ont un poids moléculaire moyen compris entre 1000 et 5500. D'un intérêt tout particulier sont les substances selon l'invention dont le poids moléculaire moyen est voisin de 1200 ± 200; cependant les substances dont le poids moléculaire moyen est voisin de 2500 ou de 5500 présentent également des propriétés intéressantes.

A titre d'enzyme protéolytique, on utilise avantageusement dans le procédé de l'invention, la trypsine, la chymotrypsine ou autre enzyme analogue, ou leurs mélanges, la trypsine étant préférée.

A titre de support approprié on peut utiliser notamment une colonne de Sephadex G 50 (marque déposée).

On indiquera à titre d'exemple non limitatif que le fractionnement, sur une colonne de Sephadex G 50, de la fraction hydrosoluble provenant du traitement de la caséine humaine délipidée et solubilisée par la trypsine pendant 24 heures à 37°C, fournit trois fractions biologiques actives, à savoir les substances IV, V et VI qui seront définies ci-après dans l'exemple 1.

On a également trouvé que la purification ultérieure de la substance V, dans des conditions appropriées, sur une colonne, de DEAE-Sephadex A-25 permet d'isoler différents produits et de sélectionner ceux dont les propriétés immunologiques sont améliorées par rapport à celles de la »substance V« elle-même.

Ainsi en fonction de la composition et du pH du solvant d'élution, on a isolé par filtration de la substance V sur DEAE-Sephadex A-25:

— une substance de caractère »basique ou neutre« appelée »MJH-24« en utilisant comme éluant une solution tampon à pH voisin de 8

— puis des substances de caractère »acide«, appelées MJH-63 et MJH-65 en utilisant comme éluant une solution tampon à un pH voisin de 3,5.

De préférence, on utilise comme solution tampon une solution de tampon »TRIS« [tris (hydroxymé-thyl) amino méthane] à différents pH, éventuellement additionnée d'un sel minéral tel que le chlorure de sodium.

La filtration sur colonne DEAE-Sephadex A-25 est suivie en mesurant l'absorption à 280 nm des fractions recueillies. Les substances actives obtenues après cette purification ultérieure sont isolées, par exemple, après dialyse puis lyophilisation de leur solution aqueuse. On a déterminé que la substance MJH-24 est un pentapeptide, qui contient les cinq acides aminés suivants: sérine, proline, leucine, phénylalanine et histidine.

Par ailleurs, les substances obtenues selon le procédé de fractionnement de la présente invention peuvent éventuellement être couplées avec des acides gras. Généralement les acides gras sont des acides aliphatiques contenant 8 à 18 atomes de carbone. D'un intérêt tout particulier est l'acide laurique. Le couplage peut s'effectuer en traitant une substance selon la présente invention par un dérivé activé d'un acide gras tel que l'anhydride ou le chlorure.

Les nouvelles substances selon la présente invention, ainsi que leurs produits de couplage avec les acides gras sont des agents immunologiques qui favorisent la production d'anticorps et que accélèrent le phénomène de phagocytose.

In vitro, ils se sont montrés particulièrement actifs à des concentrations comprises entre 0,1 et 10 µg/ml dans le test de secrétion d'anticorps (hémolytiques) anti-globules rouges de moutons par des cellules spléniques de souris immunisées in vivo et dans le test de phagocytose de globules rouges de moutons opsonisés par les macrophages péritonéaux de souris.

Les exemples suivants, donnés à titre non limitatif, montrent comment l'invention peut être mise en pratique.

### Exemple 1

La caséine humaine (400 mg) est délipidée par traitement au moyen de 25 ml d'un mélange chloroforme-méthanol (2 : 1 en volumes).

La caséine insoluble est solubilisée par traitement au moyen d'une solution d'hydroxyde de sodium 0,05 N de telle manière que la concentration finale en caséine soit voisine de 2 mg/cm$^3$.

La solution obtenue est soumise à une dialyse contre 2 litres de tampon phosphate 0,033 M à pH 8 pendant 2 jours en renouvelant 5 fois la solution tampon.

On obtient ainsi une solution à pH 8 contenant la caséine soluble. Cette solution est soumise à l'action de la trypsine de telle manière que le rapport enzyme/substrat soit voisin de 1/100. L'hydrolyse enzymatique est poursuivie pendant 24 heures à 37°C, la moitié de la quantité d'enzyme étant introduite au départ de la réaction et le reste 4 heures après le début de l'hydrolse.

Le mélange réactionnel est centrifugé à 15 000 tours/mn pendant 2 heures; le surnageant est amené à sec puis repris par 16 ml d'une solution d'acide acétique à 30%. Le mélange est soumis à 3 centrifugations successives à 13 000 tours/minute, pendant 30 minutes.

Le liquide clair surnageant (16 ml) est filtré sur une colonne de Sephadex G 50 (hauteur: 105 cm, diamètre: 2,5 cm) en éluant avec de l'acide acétique à 30% et en recueillant des fractions de 2 cm$^3$.

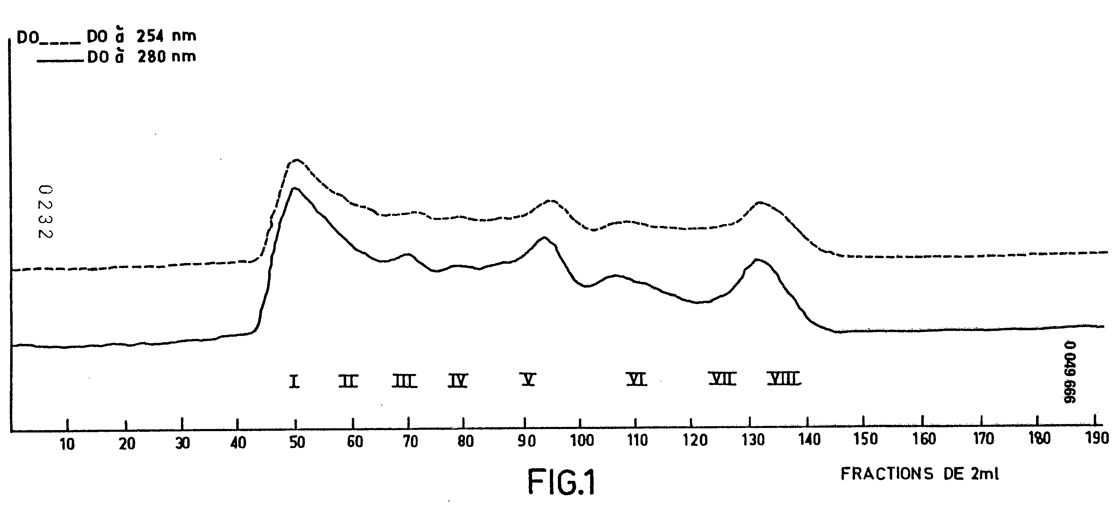

En opérant de cette manière et en suivant la chromatographie par absorption U.V. on obtient des zones qui permettent de définir 8 fractions pour lesquelles le poids moléculaire moyen est établi. Le diagramme de la filtration est représenté à la figure 1.

Les fractions bilogiquement actives sont les suivantes:

— substance IV:  fractions 71 à 82; poids moléculaire moyen 5300
— substance V:   fractions 84 à 98; poids moléculaire moyen 2230
— substance VI: fractions 99 à 114; poids moléculaire moyen 1120.

### Exemple 2

Chacune des substances obtenues à l'exemple 1 est soumise à l'action de l'anhydride laurique en opérant dans l'alcool butylique tertiaire.

Pour chacune des substances lipidées, on obtient une fraction soluble dans l'alcool butylique tertiaire et une fraction insoluble dans l'alcool butylique tertiaire.

Chacune de ces fractions est soumise aux tests biologiques: la fraction IV lipidée insoluble et la fraction VI lipidée insoluble présentent des propriétés particulièrement intéressantes.

3

### Exemple 3

467 mg de la »fraction V«, obtenus selon l'exemple 1, sont filtrés sur une colonne de DEAE-Sephadex A-25 (hauteur: 127 cm; diamètre 1,9 cm) en recueillant des fractions de 2,3 cm$^3$.

La filtration est conduite de la manière suivante:

a) élution par une solution tampon TRIS, HCl 0,01 M à pH 8: on recueille 276 fractions, parmi lesquelles les fractions 120 à 132 fournissent la substance MJH-24

b) élution par une solution tampon TRIS, HCl 0,01 M à pH 3,5 et 0,1 N en chlorure de sodium: on recueille 164 fractions qui sont éliminées (fractions 276 à 440)

c) élution par une solution tampon TRIS, HCl 0,01 M à pH 3,5 et 0,5 N en chlorure de sodium: on recueille 220 fractions (fractions 440 à 660) parmi lesquelles les fractions 543 à 553 et les fractions 571 à 604 fournissent respectivement les substances actives MJH-63 et MJH-65.

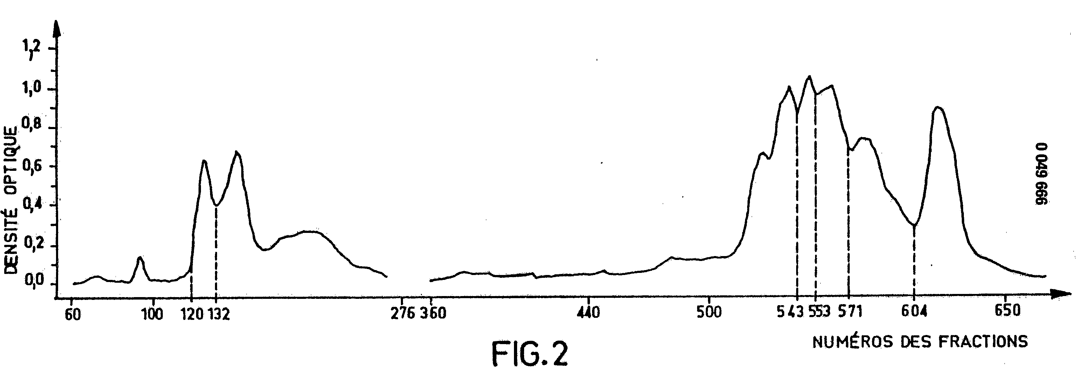

Le diagramme de filtration est représenté par la figure 2 dans laquelle figurent en abscisses les numéros des fractions et en ordonnées les densités optiques à 280 nm.

Les substances MJH-24, MJH-63 et MJH-65 ainsi obtenues présentent à un degré plus élevé, les propriétés de la substance hydrosoluble active dont elles dérivent (fraction V). In vitro elles favorisent la production d'anticorps et accélèrent le phénomène de phagocytose.

De plus, les substances MJH-24, MJH-63 et MJH-65 font preuve, chez la souris, d'une activité remarquable dès la dose de 0,05 mg/kg i.v. vis-à-vis de l'infection expérimentale à Klebsiella pneumoniae.

La présente invention concerne également les compositions utilisables en thérapeutique, qui contiennent une substance selon la présente invention en association avec un ou plusieurs diluants ou adjuvants compatibles et pharmaceutiquement acceptables.

Ces compositions peuvent être utilisées comme adjuvants de vaccins (par exemple vaccin antigrippal composé de sous-unités hémagglutinantes, vaccin antipoliomyélitique à virus inactivé, vaccin antimalarique) en injection simultanée avec l'antigène (viral, bactérien, parasitaire, fongique, tumoral) à l'égard duquel on souhaite augmenter la production d'anticorps ou la réactivité cellulaire spécifique.

Ces compositions pharmaceutiques peuvent être également employées comme immunostimulants non spécifiques en vue d'augmenter la résistance de l'hôte (homme ou animal domestique) vis-à-vis des infections ou en immunothérapie antitumorale.

En tant qu'adjuvants, les produits peuvent être administrés soit en solution aqueuse, soit en émulsion huileuse, soit encore sous forme de liposomes avec l'antigène à l'égard duquel on souhaite obtenir une réponse immunitaire augmentée ou améliorée par la voie utilisée pour cet antigène et dans des proportions variant entre 0,01 et 10 fois la quantité d'antigène avec lequel ils sont mélangés avant d'être injectés.

Pour l'application comme immunostimulant non spécifique, ces produits peuvent être utilisés par voies intraveineuse, intramusculaire, sous-cutanée, intra-nasale, éventuellement orale ou rectale, soit en solution aqueuse, soit en émulsion huileuse, soit encore sous forme de liposomes. Dans ce cas, la dose de composé selon l'invention qui est administrée est généralement comprise entre 0,01 et 25 mg/kg. En thérapeutique humaine, les doses journalières dépendent de l'effet recherché. Elles peuvent être comprises entre 0,1 et 10 mg pour un adulte.

Les compositions solides pour administration orale peuvent être des comprimés, des pilules, des poudres ou des granulés.

Comme compositions liquides pour administration orale, on peut utiliser des émulsions pharmaceutiquement acceptables, des solutions, des suspensions, des sirops ou des élixirs.

Les compositions pour administration parentérale ou intra-nasale peuvent être des solutions stériles aqueuses, des suspensions ou des émulsions.

La stérilisation peut se faire de plusieurs façons, par exemple à l'aide d'un filtre bactériologique ou en incorporant des agents stérilisants. Les compositions solides rendues stériles par irradiation (rayon $\beta$ peuvent être dissoutes dans l'eau stérile ou tout autre milieu stérile injectable éventuellement au moment de l'emploi.

Les compositions pour administration rectale sont des suppositoires.

Les exemples 4 et 5 suivants, donnés à titre non limitatif, illustrent des compositions selon l'invention.

### Exemple 4

On prépare selon la technique habituelle une composition liquide administrable par voie intraveineuse ayant la composition suivante:

— substance V obtenue à l'exemple 1    50 mg
— soluté injectable    5 ml.

### Exemple 5

On prépare selon la technique habituelle une composition liquide administrable par voie intraveineuse ayant la composition suivante:

— substance MJH-63 obtenue à l'exemple 3     50 mg
— soluté injectable     5 ml.

## Revendications

1. Procédé pour l'obtention de substances biologiquement actives, à partir de caséine humaine, caractérisé en ce que l'on traite par au moins une enzyme protéolytique de la caséine humaine délipidée et solubilisée, puis on fractionne les produits hydrosolubles, en fonction de leur poids moléculaire moyen, par filtration sur un support approprié puis on isole les substances actives, dont le poids moléculaire moyen est compris entre 1000 et 5500.

2. Procédé selon la revendication 1, caractérisé en ce que, à titre d'enzyme on utilise la trypsine, la chymotrypsine ou autre enzyme protéolytique équivalente, ou leurs mélanges, la trypsine étant préférée.

3. Procédé selon l'une des revendications 1 ou 2, caractérisé en ce que l'on traite par la trypsine de la caséine humaine délipidée et solubilisée, le rapport enzyme/substrat étant voisin de 1/100, pendant 24 heures à 37°C, puis après centrifugation, fractionne les produits hydrosolubles par filtration sur Sephadex G 50 en éluant avec de l'acide acétique à 30% puis isole lesdites substances actives, à savoir la substance IV d'un poids moléculaire moyen de 5300, la substance V d'un poids moléculaire moyen de 2230 et la substance VI d'un poids moléculaire moyen de 1120.

4. Procédé selon la revendication 1, caractérisé en ce qu'ultérieurement on filtre la substance V sur une colonne de DEAE-Sephadex A-25 en éluant avec une solution tamponnée dont le pH va en décroissant et on isole une substance de caractère basique ou neutre après élution par une solution tampon TRIS, HCl 0,01 M à pH 8 et des substances de caractère acide après élution par une solution tampon TRIS, HCl 0,01 M à pH 3,5 qui est 0,1 N en chlorure de sodium puis par une solution tampon TRIS, HCl 0,01 M à pH 3,5 qui est 0,5 N en chlorure de sodium.

5. Procédé selon la revendication 1, caractérisé en ce qu'il consiste en outre à faire réagir lesdites substances actives obtenues après filtration avec un acide gras.

6. Procédé selon la revendication 5, caractérisé en ce que l'acide gras est sous une forme activée.

7. Nouvelles substances biologiquement actives lorsqu'elles sont obtenues par le procédé selon l'une quelconque des revendications 1 à 6.

8. Nouvelles substances biologiquement actives obtenues à partir de caséine humaine par traitement à l'aide d'une enzyme protéolytique, et fractionnement, selon la revendication 7, caractérisées en ce que leur poids moléculaire moyen est respectivement de 5300 (substance IV), de 2230 (substance V) et de 1120 (substance VI).

9. Nouvelles substances biologiquement actives provenant de caséine humaine, lorsqu'elles sont obtenues par le procédé selon l'une quelconque des revendications 1 à 6.

10. Substance biologiquement active provenant de la caséine humaine constituée d'un pentapeptide de caractère basique contenant les acides aminés ci-après: sérine, proline, leucine, phénylalanine et histidine, caractérisée en ce qu'elle est obtenue par le procédé selon l'une quelconque des revendications 1 à 4.

11. Composition pharmaceutique, caractérisée en ce qu'elle contient au moins une substance selon l'une quelconque des revendications 7 à 10, en association avec un ou plusieurs diluants ou adjuvants compatibles.

## Patentansprüche

1. Verfahren zum Erhalt von biologisch aktiven Substanzen aus menchlichem Casein, dadurch gekennzeichnet, daß man lipidfrei gemachtes und löslich gemachtes menschliches Casein mit wenigstens einem proteolytischen Enzym behandelt, dann die wasserlöslichen Produkte in Abhängigkeit ihres mittleren Molekulargewichts durch Filtrieren über einen geeigneten Träger fraktioniert und dann die aktiven Substanzen, deren mittlere Molekulargewichte zwischen 1000 und 5500 liegen, isoliert.

2. Verfahren nach Anspruch 1, dadurch gekennzeichnet, daß man als Enzym das Trypsin, ds Chymotrypsin oder ein anderes äquivalentes proteolytisches Enzym oder ihre Gemische verwendet, wobei das Trypsin bevorzugt wird.

3. Verfahren nach einem der Ansprüche 1 oder 2, dadurch gekennzeichnet, daß man lipidfrei gemachtes und löslich gemachtes menschliches Casein mit Trypsin während 24 Stunden bei 37°C behandelt, wobei das Verhältnis Enzym/Substrat nahe bei 1/100 liegt, dann nach Zentrifugieren die wasserlöslichen Produkte durch Filtrieren über Sephadex G 50 fraktioniert, wobei man mit 30%iger Essigsäure eluiert, und dann die aktiven Substanzen isoliert, d. h. die Substanz IV mit einem mittleren Molekulargewicht von 5300, die Substanz V mit einem mittleren Molekulargewicht von 2230 und die Substanz VI mit einem mittleren Molekulargewicht von 1120.

4. Verfahren nach Anspruch 1, dadurch gekennzeichnet, daß man die Substanz V nachträglich über eine DEAE-Sephadex A-25 Säule filtriert, wobei man mit einer gepufferten Lösung, deren pH-Wert abnimmt, eluiert und eine Substanz von basischem oder neutralem Charakter nach Elution mit einer Tris-Pufferlösung, 0,01 M HCl bei pH 8 und Substanzen von saurem Charakter nach Elution mit einer Tris-Pufferlösung, 0,01 M HCl bei pH 3,5, die 0,1 N an Natriumchlorid ist und dann mit einer Tris-Pufferlösung, 0,01 M HCl bei pH 3,5, die 0,5 N an Natriumchlorid ist, isoliert.

5. Verfahren nach Anspruch 1, dadurch gekennzeichnet, daß man die erhaltenen aktiven Substanzen außerdem nach dem Filtrieren mit einer Fettsäure reagieren läßt.

6. Verfahren nach Anspruch 5, dadurch gekennzeichnet, daß die Fettsäure in aktivierter Form vorliegt.

7. Neue biologisch aktive Substanzen, erhalten nach dem Verfahren nach irgendeinem der Ansprüche 1 bis 6.

8. Neue biologisch aktive Substanzen, erhalten ausgehend von menschlichem Casein durch Behandlung mit einem proteolytischen Enzym und Fraktionierung nach Anspruch 7, dadurch gekennzeichnet, daß ihre mittleren Molekulargewichte 5300 (Substanz IV), 2230 (Substanz V) und 1120 (Substanz VI) betragen.

9. Neue biologisch aktive Substanzen, die aus dem menschlichen Casein stammen, erhalten nach dem Verfahren nach irgendeinem der Ansprüche 1 bis 6.

10. Biologisch aktive Substanz aus menschlichem Casein, bestehend aus einem basischen Pentapeptid, enthaltend die nachstehenden Aminosäuren: Serin, Prolin, Leucin, Phenylalanin und Histidin, dadurch gekennzeichnet, daß sie nach dem Verfahren gemäß irgendeinem der Ansprüche 1 bis 4 erhalten worden ist.

11. Pharmazeutische Zusammensetzung, dadurch gekennzeichnet, daß sie wenigstens eine Substanz nach irgendeinem der Ansprüche 7 bis 10 zusammen mit einem oder mehreren verträglichen Verdünnungsmitteln oder Hilfsstoffen enthält.

## Claims

1. Process for the preparation of biologically active substances from human casein, characterised in that solubilised human casein from which the lipids have been removed is treated with at least one proteolytic enzyme, the water-soluble products are then fractionated, according to their average molecular weight, by filtration on a suitable support, and the active substances having an average molecular weight of between 1,000 and 5,500 are then isolated.

2. Process according to Claim 1, characterised in that the enzyme used is trypsin, chymotrypsin or another equivalent proteolytic enzyme, or a mixture thereof, trypsin being preferred.

3. Process according to one of Claims 1 or 2, characterised in that solubilised human casein from which the lipids have been removed is treated with trypsin, the ratio of enzyme/substrate being of the order of 1/100, for 24 hours at 37° C, then, after centrifugation, the water-soluble products are fractionated by filtration on Sephadex G 50 using 30% acetic acid as the eluent, and the said active substances, namely the substance IV having an average molecular weight of 5,300, the substance V having an average molecular weight of 2,230 and the substance VI having an average molecular weight of 1,120, are then isolated.

4. Process according to Claim 1, characterised in that, subsequently, the substance V is filtered on a column of DEAE-Sephadex A-25 using, as the eluent, a buffered solution of decreasing pH, and a substance of basic or neutral character is isolated after elution with a 0.01 M TRIS.HCl buffer solution at pH 8, and substances of acidic character are isolated after elution with a 0.01 M TRIS.HCl buffer solution at pH 3.5 which is 0.1 N in respect of sodium chloride, and then with a 0.01 M TRIS.HCl buffer solution at pH 3.5 which is 0.5 N in respect of sodium chloride.

5. Process according to Claim 1, characterised in that it also consists in reacting the said active substances, obtained after filtration, with a fatty acid.

6. Process according to Claim 5, characterised in that the fatty acid is in activated form.

7. New biologically active substances when they are obtained by the process according to any one of Claims 1 to 6.

8. New biologically active substances obtained from human casein by treatment with a proteolytic enzyme and fractionation, according to Claim 7, characterised in that their average molecular weight is respectively 5,300 (substance IV), 2,230 (substance V) and 1,120 (substance VI).

9. New biologically active substances originating from human casein, when they are obtained by the process according to any one of Claims 1 to 6.

**049 666**

10. Biologically active substance originating from human casein consisting of a pentapeptide of basic character containing the following aminoacids: serine, proline, leucine, phenylalanine and histidine, characterised in that it is obtained by the process according to any one of Claims 1 to 4.

11. Pharmaceutical composition, characterised in that it contains at least one substance according to any one of Claims 7 to 10, in association with one or more compatible diluents or adjuvants.

7

FIG.1

FRACTIONS DE 2ml

FIG.2

DENSITÉ OPTIQUE

NUMÉROS DES FRACTIONS